# EUROPÄISCHE PATENTSCHRIFT

(11) **EP 1 941 897 B1**
(45) Veröffentlichungstag und Bekanntmachung des Hinweises auf die Patenterteilung: **09.12.2009**
(21) Anmeldenummer: 07115510.5
(22) Anmeldetag: 03.09.2007
(51) Int. Cl.: A61K 38/08, A61P 17/00

(54) **Oligopeptide enthaltende dermatologische Zusammensetzungen zur Steigerung der Hautempfindlichkeit**
Dermatological compounds containing oligopeptides for increasing skin sensitivity
Compositions dermatologiques contenant des oligopeptides afin d'augmenter la sensibilité de la peau

(30) Priorität: 07.10.2006 DE 102006047529
(43) Veröffentlichungstag der Anmeldung: 09.07.2008
(73) Patentinhaber: Evonik Goldschmidt GmbH, 45127 Essen (DE)
(72) Erfinder: Lersch, Peter, 46537, Dinslaken (DE); Farwick, Mike, 45138, Essen (DE)

(56) Entgegenhaltungen:
- DE-A1-102005 063 179
- US-A1- 2004 132 667
- DATABASE WPI Week 200244 Derwent Publications Ltd., London, GB; AN 2002-410928 XP002482368 & JP 2002 080497 A (MERCIAN CORP) 19. März 2002 (2002-03-19)
- YANAI T ET AL: "Prolyl Endopeptidase Inhibitory Peptides in Wine" BIOSCI BIOTECHNOL BIOCHEM, Bd. 67, Nr. 2, 2003, Seiten 380-382, XP002482367

## Beschreibung

Die Erfindung betrifft dermatologische Zusammensetzungen und die Verwendung von spezifischen Oligopeptiden in solchen dermatologischen Zusammensetzungen zur Steigerung der Hautempfindlichkeit bei reduzierter oder verlorener Wahrnehmungskraft über das Sinnesorgan Haut.

Die Haut (Cutis) ist mit ca. 2 qm² das größte und nervenreichste Organ des menschlichen Körpers und erfüllt mit ihrem Schichtenaufbau aus Epidermis (Oberhaut), Dermis (Corium) und Subcutis (Unterhaut) zahlreiche Aufgaben. Sie schützt den gesamten Organismus vor Krankheitserregern oder Molekülen, Sonneneinstrahlung und Austrocknung. Daneben werden über Hautporen aber Sauerstoff und Nährstoffe aufgenommen. Die Haut dient zudem der Regulierung und Erhaltung der Körpertemperatur.

Die menschliche Haut erlangt durch eine Vielzahl verschiedener Rezeptoren - etwa 7 bis 50 pro cm² - die Fähigkeit zur Aufnahme verschiedenster Sinneseindrücke, von Temperaturempfindungen über Schmerz und Juckreiz bis hinzu leichten Berührungen. Auch koordinative Informationen wie die Stellung der Körperteile im Raum werden über Hautrezeptoren erfasst. Dabei werden von den speziellen Rezeptoren physikalische bzw. chemische Reize, die auf den Organismus einwirken, registriert und die Information in Form neuronaler Aktivität über das periphere Nervensystem ans Gehirn geleitet. In der Epidermis wurden beispielsweise die Merkel-Zellen als Mechanorezeptoren für Tast- und Druckempfindungen identifiziert. Weitere wichtige Sensoren sind die Vater-Pacinische-Körperchen im Subcutisbereich, die auf Vibrationen reagieren und die Meissnersche Tastkörperchen oder Tastscheiben an der epidermalendermalen Grenze, die für Berührungsempfindungen zuständig sind. Weitere ausgebildete Sinnesorgane im Bereich der Dermis sind die Ruffinischen Körperchen (Dehnungsrezeptoren) sowie die Krause Körperchen oder Endkolben (Mechanorezeptoren). Intraepitheliale Nervenendungen sind sensible Nervenfasern in der Haut, die Wärme-, Kälte-, Schmerz-, Berührungs- sowie Druckempfindungen auslösen.

Ein Nozizeptor (lat. nocere: schaden) ist ein Rezeptor, der auf eine drohende oder eingetretene Verletzung des Körpergewebes reagiert. Die Dichte der Nozizeptoren beim Menschen ist größer als die aller anderen Hautrezeptoren. Die Verteilung auf der Körperoberfläche ist relativ gleichmäßig. Die Veränderung der Reizschwelle wird durch die Freisetzung körpereigener, chemischer Substanzen bewirkt, die die Nozizeptoren empfindsamer und aufnahmefähiger machen. Einige dieser Substanzen sind Bradykinin, Prostaglandin E2 und Serotonin. Bei einer Schädigung (Noxe) werden diese Substanzen freigesetzt. Steigt die Konzentration dieser Stoffe über ein gewisses Maß an, so kommt es zu einer Schmerzreaktion. Aber auch, wenn aufgrund der Konzentration die Schmerzschwelle nicht überschritten wird, reicht eine geringe Menge dieser Stoffe aus, um die Erregbarkeit der Nozizeptoren zu steigern. Die Nozizeptoren sind dann empfindlicher und der Mensch nimmt Schmerzen eher und stärker wahr. Durch die Schmerzstoffe wird nicht nur eine Sensibilisierung der Nervenenden bewirkt. Diese Stoffe wirken sich auch auf die Durchlässigkeit der Gefäßwände aus und bewirken ein Engstellen der Blutgefäße. Die gesamte chemische Umgebung des Nozizeptors wird verändert und steigert seine Erregbarkeit.

Insbesondere Peptide erfüllen im menschlichen Körper und auch in der Haut wichtige Aufgaben als bioaktive Botenstoffe, beispielsweise als Hormone, Neurotransmitter oder als Neuromodulatoren. Die Substanzklasse der Neuropeptide spielt dabei eine wesentliche Rolle, denn hierbei handelt es sich um kurze Oligopeptide, die oft eine Zwischenstellung zwischen Hormonen und reinen Neurotransmittern einnehmen. Ihre Funktion wird auch als neuromodulatorisch bezeichnet, da sie die Wirkung von anderen Transmittern graduell unterstützen oder hemmen können. Die Synthese und der Transport sind im Gegensatz zu kleinmolekularen Transmittern eher langsam und träge. Neuropeptide binden zudem nicht direkt an Ionenkanäle und verändern somit auch nicht die Spannung der postsynaptischen Membran, sondern wirken über Rezeptoren auf Zellfunktionen und auf die Zellstruktur der postsynaptischen Zielzelle.

Es gibt eine Vielzahl von wissenschaftlichen Untersuchungen, die sich auf die Rolle von Oligopeptiden als Botenstoffe und als Neuromodulatoren im Gehirn konzentrieren. So wurde bei immunologischen Untersuchungen festgestellt, dass bei Reizung sensorischer Nervenendungen Neuropeptide ausgeschüttet werden, die wesentlich für die neuronale Wahrnehmung und Reizweiterleitung im Gehirn sind. Substanz P ist ein Beispiel für ein solches Neuropeptid, welches vor allem als Schmerz- und Entzündungsmediator bekannt geworden ist. Es besteht aus 11 Aminosäuren und gehört zur Gruppe der Neurokinine und wird von Nervenzellen, aber auch von Leukozyten gebildet. Die Aminosäurensequenz ist Arg - Pro - Lys - Pro - Gln - Gln- Phe - Phe - Gly - Leu - Met - NH2.

Wird ein afferenter Nozizeptor stärker erregt, setzt er Substanz P frei, die zunächst als Neurotransmitter bei Schmerzrezeptoren und schmerzleitenden C-Fasern erkannt wurde. Substanz P spielt aber auch als Modulator bei Entzündungsreaktionen eine Rolle und bewirkt eine starke Erweiterung der Blutgefäße und steigert die Durchlässigkeit der Gefäßwände. Als Folge davon wird die örtliche Durchblutung des Gewebes erhöht. Auch diese Vorgänge bewirken eine Steigerung der Empfindsamkeit des Nozizeptors.

Auch die Wirkung des in Paprika und Chili natürlich vorkommenden Alkaloids Capsaicin, bei dem es sich chemisch um ein Vanillylamid der 8-Methyl-6-nonensäure handelt, beruht auf der heftigen Stimulation von Chemo-Nozizeptoren in Nervenzellen. Capsaicin reizt die Nervenenden bestimmter Nozizeptoren, die normalerweise Schmerzreize bei Einwirkung von Hitze oder chemischer Reizung erkennen. Capsaicin bindet an den TRP-Kanal *TRPV1,* der auch durch eine Erhöhung der Temperatur aktiviert wird. Der schmerzhaften (aber nur scheinbaren) Erhitzung durch Capsaicin wirkt der Organismus durch Ausschüttung von Substanz P aus intrazellulären Speichervesikeln entgegen. Dies führt akut zu einer Membrandepolarisierung und durch die vermehrte Durchblutung des Gewebes zum Zweck der Wärmeabfuhr kommt es zu einer lokalen Rötung, wie bei einer leichten Verbrennung.

Substanz P-Antagonisten sind augenblicklich stark im Fokus der wissenschaftlichen Forschung, beispielsweise für die Schmerztherapie und als potentielle Antidepressiva.

Die Wirkung von Substanz P im synaptischen Spalt wird durch raschen Peptidase-vermittelten Abbau beendet. Schutz vor dem Abbau durch verschiedene Endopeptidasen kann durch Enzyminhibitoren erreicht werden. Dieses Phänomen ist Gegenstand aktueller pharmazeutischer Untersuchungen. Verschiedene Substanzklassen werden zu diesem Zweck untersucht. Neben anderen Oligopeptiden wurden bei neueren medizinischen Untersuchungen zwei prolinhaltige Pentapeptide mit der Sequenz Val-Glu-Ile-Pro-Glu und Tyr-Pro-Ile-Pro-Phe identifiziert. Es wird vermutet, dass diese in der Lage sind, Prolyl-Endopeptidase zu inhibieren.

Dieses Enzym ist vermutlich in der Pathogenese der Alzheimer-Krankheit und Senilität über den Abbau verschiedener Neuropeptide beteiligt.

So werden beispielsweise von Yanai, Sato und Suzuki in Biosci. Biotechnol. Biochem., 67 (2), 380 bis 382, 2003, Oligopeptide mit den Sequenzen Val-Glu-Ile-Pro-Glu und Tyr-Pro-Ile-Pro-Phe offenbart, die geeignet sind, die Prolylendpeptidase zu inhibieren.

Die Verwendung von bestimmten Peptiden und Oligopeptiden als Mittel gegen äußere Anzeichen der Hautalterung in Hautpflegeprodukten ist beispielsweise aus den nachstehenden Anmeldungen bekannt.

So offenbart die WO 2005/048968 die Verwendung einer Kombination von Tri- und Tetrapeptiden in kosmetischen Zusammensetzungen, um topisch sichtbare Hautveränderungen, wie Falten und dunkle Ringe, zu mildern oder zu vermeiden. Die Kombination der in der WO 2005/048968 beschriebenen Peptide soll eine vermehrte Produktion von Collagen I, Fibronectin, Collagen IV und Hyaluronsäure in Hautzellen bewirken.

Die WO 2003/7068141 offenbart eine kosmetische oder dermopharmazeutische Zusammensetzung zur Verminderung von Symptomen der Hauterschlaffung und Hautalterung. Die beanspruchte Zusammensetzung enthält eine synergistische Kombination von mindestens zwei, bevorzugt drei Komponenten die ausgewählt sind aus
a) Hesperidin oder eines Derivats hiervon,
b) A.C.E. Enzyminhibitor-Dipeptiden und
c) Oligopeptiden R₂-(AA)n-Pro-Arg-OH, wobei (AA)ₙ eine Peptidkette aus Aminosäuren oder Derivaten davon ist, wobei n zwischen 1 und 3 und R₂ = H oder eine Alkylkette mit C₂ bis C₂₂ ist.

In der US 5,492,894 wird ein Oligopeptid mit 3 bis 6 Aminosäuren in einer topischen Zusammensetzung zur kosmetischen Behandlung von Falten verwendet, wobei drei der Aminosäuren unabhängig voneinander aus Lys oder Arg ausgewählt sind.

In neueren kosmetischen Formulierungen zur topischen Applikation auf der Haut werden Oligopeptide eingesetzt, die eine muskelentspannende Wirkung zeigen und somit Mimikfalten, wie sogenannte Krähenfüße, in der Haut vermindern oder beseitigen können. Ein Beispiel hierfür ist ein Hexapeptid, das Acetyl Hexapeptid-3 (Argireline). Es kann das Nervensignal zur Muskelkontraktion, nämlich den Botenstoff Acetylcholin, hemmen und somit durch die Unterbrechung der Reizweiterleitung die Haut oberflächlich entspannen und glätten. Solche Formulierungen werden als Alternative zu Antifalten-Behandlungen mit Injektionen des Nervengifts Botulinumtoxin A (Botox) eingesetzt.

Die Formulierungen der im Stand der Technik bekannten kosmetischen oder dermatologischen Hautpflegeprodukte zielen jedoch alle auf die Verbesserung des äußeren Erscheinungsbildes der alternden Haut ab.

Keine der beschriebenen Substanzen oder Formulierungen unterstützt oder verbessert die Haut in ihrer Funktion als Sinnesorgan, wie beispielsweise durch die Verbesserung der neuronalen Reizwahrnehmung über die Haut.

Die Einschätzung, ob Alterungsphänomene der Haut eher als Konsequenz eines physiologischen Prozesses oder als Krankheitszustand wahrzunehmen sind, fällt je nach kulturellem, sozialem und psychologischem Hintergrund unterschiedlich aus. Zu den charakteristischen Merkmalen der alternden Haut zählen Atrophie, Trockenheit, Rauhigkeit, Faltenbildung, Elastizitätsverlust, Pigmentunregelmäßigkeiten, eine Neigung zur Ausbildung multipler, meist gutartiger Neubildungen und eine erhöhte Verletzlichkeit als Folge tief greifender struktureller und funktioneller Veränderungen, die letztendlich zu einer Verdünnung der Hautschichten und einer Verminderung ihrer funktionstragenden Komponenten führen. Auch die neuronale Wahrnehmung der Haut wird durch Alterungsvorgänge entscheidend beeinträchtigt. So ist beispielsweise die Thermoregulation bei der alten Haut zunehmend träger, d.h. die Schutzorganismen des Körpers vor Wärmeverlust oder Überhitzung funktionieren nicht mehr in dem Maße, wie man es von der jungen Haut gewohnt war. Dies liegt an der sich zunehmend mindernden Fähigkeit der Gefäße sich zu erweitern bzw. zu verengen.

Aufgabe der vorliegenden Erfindung ist daher die Bereitstellung von dermatologischen Zusammensetzungen und deren Verwendung zur Steigerung der Hautempfindlichkeit bei reduzierter oder verlorener Wahrnehmungskraft über das Sinnesorgan Haut.

Es wurde überraschenderweise gefunden, dass diese Aufgabe durch die Verwendung von mindestens einem Oligopeptid mit einer Sequenz Ala-IIe-Pro-Gln, Arg-Lys-Pro-Ile-Pro, Palmitoyl-Gly-His-Pro-Ile-Ser, Gly-Pro-Ile-Pro, Gly-Val-Glu-Ile-Pro-Ala, Pro-Ile-Pro-Phe, Octanoyl-Ser-His-Pro-Ile, Hexanoyl-Thr-Lys-Pro-Ile-Pro-Arg, Tyr-Pro-Ile-Ser-Leu, Val-Glu-Ile-Pro-Glu oder Tyr-Pro-Ile-Pro-Phe gelöst werden kann.

Ein Gegenstand der Erfindung sind daher dermatologische Zusammensetzungen enthaltend mindestens ein Oligopeptid mit einer Sequenz Ala-IIe-Pro-Gln, Arg-Lys-Pro-Ile-Pro, Palmitoyl-Gly-His-Pro-Ile-Ser, Gly-Pro-Ile-Pro, Gly-Val-Glu-Ile-Pro-Ala, Pro-Ile-Pro-Phe, Octanoyl-Ser-His-Pro-Ile, Hexanoyl-Thr-Lys-Pro-Ile-Pro-Arg, Tyr-Pro-Ile-Ser-Leu, Val-Glu-Ile-Pro-Glu oder Tyr-Pro-Ile-Pro-Phe und/oder ein Acyl-Derivat hiervon zur Verwendung als Mittel zur Steigerung der Hautempfindlichkeit bei reduzierter oder verlorener Wahrnehmungskraft über das Sinnesorgan Haut, weiterhin als die Zusammensetzung nach Anspruch 1 genannt.

Es wird im Rahmen der Beschreibung der Erfindung der Dreibuchstaben-Code für die Aminosäuren verwendet, wobei in Sequenzen grundsätzlich von links nach rechts zunächst das N-terminale Ende und dann das C-terminale Ende angegeben wird. Dies entspricht der allgemein üblichen Kurzschreibweise der Aminosäuren, wie sie zum Beispiel in BIOCHEMIE; Berg, J. M., Tymoczko, J. L., Stryer L.; 5. Auflage; Spektrum Akademischer Verlag; Heidelberg Berlin; 2003, S. 55; Tabelle 3.2, wiedergegeben ist.

Die erfindungsgemäßen Oligopeptide sind für die topische Applikation auf die Haut geeignet. Sie werden gut von der menschlichen Haut toleriert und können eine gute Bioverfügbarkeit aufweisen. Sie können eine gute Wirksamkeit zur Steigerung der Hautempfindlichkeit.

Gerade im Hinblick auf die Regeneration von reduzierter oder verlorener Wahrnehmungskraft über das Sinnesorgan Haut, beispielsweise durch Schädigung oder Vorerkrankungen, besteht ein Bedarf an neuen, wirksamen dermatologischen Zusammensetzungen. Eine geschwächte oder gestörte Sinneswahrnehmung über die Haut kann das Wohlbefinden, beispielsweise durch ein Taubheitsgefühl, nachhaltig negativ beeinflussen.

Vorteilhafterweise können die erfindungsgemäßen Oligopeptide in dermatologischen Zusammensetzungen zur topischen Applikation bei Menschen eingesetzt werden, deren Reizwahrnehmung über die Haut beispielsweise durch Schädigung oder Vorerkrankungen reduziert, gestört oder zumindest zeitweise verloren ist. Dies können beispielsweise Patienten mit Narben, Verbrennungen, Verletzungen sein. Es ist auch bekannt, dass bei Schlaganfallpatienten die Reizwahrnehmung, also beispielsweise Wärme- und Kälteempfinden, über die Haut zumindest vorübergehend ausfallen kann.

Die Verwendung der erfindungsgemäßen Oligopeptide in dermatologischen Zusammensetzungen kann hierbei eine schnellere Wiederherstellung der Reizempfindungen über die Haut bewirken und somit weiteren Schädigungen der Haut vorbeugen. Diese Wiederherstellung des Hautempfindens und der Wahrnehmung trägt zudem erheblich zu einem verbesserten Allgemeinempfinden und zu einer schnelleren Rehabilitation solcher Patienten bei.

Die Oligopeptide können einfach und kostengünstig über die klassischen Wege der peptidischen Synthese, beispielsweise durch automatisierte Festphasensynthese an Merrifield-Harzen oder über enzymatische Wege in der erforderlichen Reinheit, gewonnen werden. Ebenso weiß der Fachmann auf dem Gebiet der peptidischen Synthese, wie erfindungsgemäße Derivate der Oligopeptide mit bekannten Methoden erzeugt werden können.

Das Oligopeptid oder Acyl-Derivat weist erfindungsgemäß eine natürlich vorkommende Aminosäuresequenz auf. Im Sinne der Erfindung bedeutet "natürlich vorkommend", dass die Aminosäuresequenz des Oligopeptids vollständig oder als bioaktiver Teil einer Gesamtsequenz in der Natur oder in Naturprodukten, beispielsweise in Weintrauben und in Wein oder Saft aus Weintrauben, vorkommt. Die erfindungsgemäßen Oligopeptide können nach ihren natürlichen Vorbildern synthetisch hergestellt oder direkt aus den entsprechenden natürlichen Quellen, beispielsweise durch Extraktion, gewonnen werden.

Ein Vorteil hierbei ist, dass diese erfindungsgemäß eingesetzten Oligopeptide eine besonders gute Aktivität und Bioverfügbarkeit aufweisen können und grundsätzlich gut verträglich, toxikologisch unbedenklich und biologisch abbaubar sind.

Zur Steigerung der Hautempfindlichkeit und der neuronalen Wahrnehmung hat sich die Verwendung des Pentapeptids mit der Aminosäuresequenz Val-Glu-Ile-Pro-Glu und/oder Tyr-Pro-Ile-Pro-Phe herausgestellt. Diese erfindungsgemäßen Oligopeptide zeigen eine besonders gute aktivierende, stimulierende Wirkung für die Reizwahrnehmung über die Haut.

Zur Acyl-Derivatisierung der erfindungsgemäß verwendeten Oligopeptide durch Amidverbindung eine alkylische lipophile Kette oder ein arylischer Rest oder deren alkyloxische oder aryloxische oder alkylaryloxische Variante an das N-terminale Ende des Oligopeptids und/oder am C-terminalen Ende durch Esterverbindung ein alkylischer Alkohol oder durch Amidverbindung eine NH₂- oder eine solche N-alkylisch substituierte Gruppe angebracht werden.

Erfindungsgemäß kann eine Acylgruppe am N-terminalen Ende der Aminosäuresequenz angeordnet sein. Diese kann optional verzweigte oder geradkettige, lang- oder kurzkettige, gesättigte oder ungesättigte Reste tragen, unsubstituiert oder mit einer oder mehreren Hydroxyl-, Amino-, Acylamino-, Sulfat- oder Sulfidgruppen substituiert sein. Solche N-acylischen Derivate können beispielsweise mit Essigsäure, Biotinsäure, Caprylsäure, Caprinsäure, Laurinsäure, Myristinsäure, Oktansäure, Palmitinsäure, Stearinsäure, Behensäure, Linolsäure, Linolensäure, Liponsäure, Ölsäure, Isostearinsäure, Elaidinsäure, 2-Ethylhexansäure, Kokosnussölfettsäure, Talkfettsäure, gehärtete Talkfettsäure, Palmkernöl-Fettsäure, Lanolin-Fettsäure oder Mischungen hiervon erzeugt werden.

Bevorzugte Acylgruppen umfassen substituierte oder unsubstituierte Acetyl-, Palmitoyl- Hexanoyl-, Myristyl-, Biotinyl und Oktanoylgruppen.

Erfindungsgemäß geeignete Peptidsequenzen umfassen:
Ala-IIe-Pro-Gln
Gly-Pro-Ile-Pro
Octanoyl-Ser-His-Pro-Ile
Arg-Lys-Pro-Ile-Pro
Tyr-Pro-Ile-Ser-Leu
Palmitoyl-Gly-His-Pro-Ile-Ser
Gly-Val-Glu-Ile-Pro-Ala
Hexanoyl-Thr-Lys-Pro-Ile-Pro-Arg.

Besonders bevorzugt im Sinne der Erfindung sind die N-Palmitoyl-Derivate, insbesondere N-Palmitoyl-Val-Glu-Ile-Pro-Glu und/oder N-Palmitoyl-Tyr-Pro-Ile-Pro-Phe.

Ein besonderer Vorteil ist, dass durch eine solche Abwandlung und Derivatisierung die Lipophilie des oligopeptids erhöht werden und somit eine höhere Affinität zur Oberhaut und ein-deutlich verbessertes Penetrationsvermögen erhalten werden kann. So kann die biologische Wirkung des Oligopeptids erheblich gesteigert werden.

Die Aufgabe der vorliegenden Erfindung wird durch die Zusammensetzung nach Anspruch 1 gelöst.

Die Zusammensetzung nach Anspruch 1 umfasst bevorzugt pharmazeutische Zusammensetzungen und Arzneimittel, die bevorzugt topisch angewendet wird. Optional können die dermatologischen Zusammensetzungen gleichzeitig mehrere Zwecke erfüllen und auch kosmetischen Zwecken und/oder zur Bereitstellung eines oder mehrerer zusätzlicher pharmazeutischer Mittel dienen.

Als besonders wirkungsvoll zur Stimulierung der Reizwahrnehmung in der Haut haben sich die Pentapeptide mit der Aminosäuresequenz Val-Glu-Ile-Pro-Glu und/oder Tyr-Pro-Ile-Pro-Phe herausgestellt, die beispielsweise im Wein enthalten sein können.

Die Zusammensetzung nach Anspruch 1 kann AcylDerivate, des erfindungsgemäß geeigneten Oligopeptids enthalten. Besonders bevorzugt ist hierbei ein erfindungsgemäß geeignetes Oligopeptid, das mit substituierten oder unsubstituierten Acetyl-, Hexanoyl-, Myristyl-, Palmitoyl- und/oder Biotinyl-Gruppen derivatisiert ist.

Durch eine solche Abwandlung und Derivatisierung kann die Lipophilie des Oligopeptids erhöht werden, um so eine höhere Affinität zur Oberhaut und ein deutlich verbessertes Penetrationsvermögen zu erhalten. Durch eine solche Maßnahme kann die biologische Wirkung des erfindungsgemäß geeigneten Oligopeptids erheblich gesteigert werden. Als besonders wirkungsvoll zur Stimulierung der Reizwahrnehmung hat sich die Verwendung der Pentapeptide mit der Aminosäuresequenz N-Palmitoyl-Val-Glu-Ile-Pro-Glu und/oder N-Palmitoyl-Tyr-Pro-Ile-Pro-Phe herausgestellt.

Ein Vorteil hierbei ist, dass die notwendige Konzentration an Oligopeptid, um die gewünschte Wirkung zu erzielen, in der dermatologischen Zusammensetzung durch die verbesserten Eigenschaften deutlich verringert werden kann.

Die Zusammensetzung nach Anspruch 1 enthält mindestens ein Oligopeptid und/oder Derivat hiervon in einer Wirkstoff-Menge, die zwischen 0,0005 Gew.-% und 2 Gew.-%, bevorzugt zwischen 0,01 Gew.-% und 1 Gew.-%, und weiter bevorzugt zwischen 0,05 Gew.-% und 0,5 Gew.-%, bezogen auf das Gewicht der Gesamtzusammensetzung, liegt.

Grundsätzlich wird die eingesetzte Menge des Oligopeptids als Wirkstoff in der Gesamtzusammensetzung so bemessen, dass die gewünschte Steigerung der Hautempfindlichkeit erzielt werden kann.

Sofern nichts anderes angegeben ist, erfolgen die Mengenangaben innerhalb der vorliegenden Beschreibung in Gewichtsprozent (Gew.-%) in Bezug auf die Gesamtmenge der dermatologischen Zusammensetzung.

Die Zusammensetzung nach Anspruch 1 enthält die dermatologische Zusammensetzung das Oligopeptid und/oder ein Derivat hiervon in einer geeigneten Trägersubstanz oder einem geeigneten Trägersystem aus mehreren Trägersubstanzen.

Die dermatologischen Zusammensetzungen können beispielsweise als Öl-in-Wasser-(O/W-)-Emulsionen, Wasser-in-Öl-(W/O)-Emulsionen, Wasser-in-Silikon-Emulsionen, Mehrfachemulsionen (beispielsweise W/O/W oder O/W/O), Öl-in-Wasser-in-Silikon-Emulsionen, Mikroemulsionen, Nanoemulsionen, PIT-Emulsionen, Pickering-Emulsionen, Suspensionen, Dispersionen, Hydrogele, Lotionen, Gele, Lipogele, ein- oder mehrphasige Lösungen, Schäume, Pflaster, Cremes, Salben, Pasten, Puder und/oder als andere übliche topische Mittel formuliert sein, die beispielsweise auch über Stifte, Masken oder als Sprays verabreicht werden können.

Zusätzlich können zu den Oligopeptiden und/oder ihren Acyl-Derivaten oder Mischungen hieraus verschiedene andere und zusätzliche aktive und/oder funktionelle Bestandteile enthalten sein, die üblicherweise in topischen und/oder transdermalen pharmazeutischen und/oder kosmetischen Hautprodukten oder Körperpflegeprodukten eingesetzt werden.

Dies können beispielsweise Fette, Öle, Wachse, Silikone, Emulgatoren, Alkohole, Polyole, Verdickungsmittel, Quellmittel, Strukturgeber, Konsistenzgeber, anfeuchtende oder feuchthaltende Substanzen, Tenside, weichmachende Substanzen (Emollients), Schaumbremsen, anionische, kationische oder amphotere Polymere, Alkalisierungs- oder Acidifizierungsmittel, Puffersubstanzen, pH-Wert-Regler, Stabilisatoren, Enthärter, Adsorbentien, Adstringenzien, Lichtschutzmittel, Filmbildner, Elektrolyte, Sequestrierungsmittel, Wasser, organische Lösungsmittel, Konservierungsstoffe, Stabilisatoren, antimikrobielle und biozide Substanzen, Bakterizide, Antioxidantien, Pharmazeutika, Vitamine, Komplexbildner, Enzyme, Duftstoffe, Aromen oder Farbstoffe sein. Diese Aufzählung ist nur beispielhaft und nicht als abschließend anzusehen.

Weitere Bestandteile und Substanzen für dermatologische Zusammensetzungen sind dem Fachmann bekannt und können dem Stand der Technik, beispielsweise auch der WO 2005/048968 entnommen werden.

Ein erfindungsgemäß geeignetes Oligopeptid wird bevorzugt mit einem oder mehreren dermatologisch verträglichen Zusatz- und Hilfsstoffen formuliert. Hierunter werden erfindungsgemäß pharmazeutisch und kosmetisch verträgliche Substanzen verstanden, die zur Verwendung im Kontakt mit der menschlichen Haut geeignet sind, unter anderem in Bezug auf Toxizität, Unverträglichkeiten, Hautirritationen und allergische Reaktionen.

Es werden erfindungsgemäß vorzugsweise solche Zusatz- und Hilfsstoffe ausgewählt, die die gewünschte Wirkung der Oligopeptide in der dermatologischen Zusammensetzung und/oder ihre Applizierbarkeit positiv beeinflussen. Vorteilhafterweise lassen sich die erfindungsgemäßen Oligopeptide gut mit üblichen Zusatz- und Hilfsstoffen kombinieren und können problemlos in übliche und/oder zugelassene dermatologische Rahmenrezepturen eingearbeitet werden.

Die Oligopeptide und/oder ihre AcylDerivate können vor Einbringung in die Gesamtzusammensetzung in Lösung gebracht werden. Die Peptide lassen sich beispielsweise im Allgemeinen in klassischen kosmetischen oder hautpharmakologischen Lösungsmitteln wie Ethanol, Propanol oder Isopropanol, Glykol-Propylen, Glyzerin, Butylen-Glykol, Ethoxy-Diglykol, Polyethylen-Glykol; Methylethern oder Diglykolen-Ethylenen, zyklischen Polyolen, ethoxylische oder propoxylische Diglykolen oder beliebigen Mischungen dieser Lösungsmittel lösen. Methoden zur Solubilisierung von Oligopeptiden sind dem Fachmann bekannt. Die Oligopeptide oder ihre Derivate können auch vorab in dermato-pharmazeutische oder kosmetische Vektoren (Transportsysteme), beispielsweise Liposome, Chylomikrone, Makro, Mikro- oder Nanopartikel sowie Makro-, Mikro- oder Nanokapseln, eingebracht oder verkapselt werden oder auf pulvrigen, organischen Polymeren, Talken, Bentonit, Siliciumdioxid oder anderen mineralischen Medien absorbieren oder adsorbieren.

Diese Lösungen oder Zubereitungen lassen sich anschließend in allen möglichen galenischen, hautpharmakologischen Formulierungen einsetzen.

Beispielsweise kann durch die Erzeugung von O/W-Nanoemulsionen als kolloidales Trägersystem bei schwer wasserlöslichen Arzneimitteln die Penetration und Permeation durch die Haut verbessert und damit die Bioverfügbarkeit der Wirkstoffe gesteigert werden.

Die Zusammensetzung nach Anspruch 1 kann zudem eine wirksame Menge eines Penetrationsbeschleunigers, eines sogenannten "Enhancers", enthalten, der in der Lage ist, den Wirkstofftransport durch und/oder in die Haut zu fördern.

Lösungsmittel wie Alkohole, Alkylmethylsulfoxide und Polyole erhöhen hauptsächlich die Löslichkeit und beeinflussen den Verteilungskoeffizienten günstig. Außerdem können einige Lösungsmittel wie Dimethylsulfat (DMSO) oder Ethanol epidermale Lipide extrahieren und so die Permeabilität der Hornschicht erhöhen. Ölsäure, Azone (epsilon-Laurocapram) und Isopropylmyristat sind typische Beispiele für Penetrationsbeschleuniger, die interkalierend in die Lipiddoppelschichten der Haut eingebaut werden und deren dichte Packung stören, wodurch die Diffusionsgeschwindigkeit gesteigert werden kann. Ionische Tenside, Decylmethylsulfoxid, DMSO oder Harnstoff lockern die feste Proteinstruktur in den Hornzellen auf und der Diffusionskoeffizient steigt.

Es werden im Folgenden Beispiele für oben genannte zusätzliche dermatologische Wirk-, Zusatz- und Hilfsstoffe beschrieben, die in Kombination mit den Oligopeptiden in der Zusammensetzung nach Anspruch 1 eingebracht werden können. Beispiele im Rahmen der Beschreibung dienen nur der weiteren Erläuterung der Erfindung und sind für diese nicht limitierend.

Die beschriebenen Stoffe und Substanzen können in den dermatologischen Zusammensetzungen der Erfindung eine oder auch mehrere Funktionen erfüllen und/oder eine oder mehrere pharmazeutische und/oder kosmetische Wirkungen haben.

Die Zusammensetzung nach Anspruch 1 kann in vorteilhafter Weise zur zusätzlichen Pflege und zum Schutz der Haut Kreatin, Ceramide, Pseudoceramide, Proteinhydrolysate pflanzlichen oder tierischen Ursprungs auf Basis Keratin, Collagen, Elastin, Weizen, Reis, Soja, Milch, Seide, Mais, Aminosäure und Aminosäurederivate, Polyasparaginsäure(derivate), entzündungshemmende Wirkstoffe, antimikrobielle Wirkstoffe, gebräuchliche Antioxidantien, Vitamine, Dexpanthenol, Retinol, Niacinamid, Milchsäure, Pyrrolidoncarbonsäure, Bisabolol sowie Pflanzen-, Hefe- und Algenextrakte enthalten.

Diese können in Synergie mit den erfindungsgemäßen Oligopeptiden beispielsweise eine wundheilungsfördernde, feuchtigkeitsspendende Anti-Falten- oder Anti-Cellulite-Wirkung haben oder als hautpflegende Bestandteile eingesetzt werden.

Solche Zusammensetzungen können beispielsweise in der Nachbehandlung von Wunden und Narben eingesetzt werden und zusätzlich neben der Wiederherstellung und/oder Verbesserung des Hautempfindens und der neuronalen Wahrnehmung von Reizen über die Haut durch die Wirkung der erfindungsgemäßen Oligopeptide auch gute Ergebnisse im Hinblick auf die Heilung und Wiederherstellung des Hautgewebes erzielen.

Diese Zusammenwirkung kann besonders vorteilhaft sein, wenn das Hautgewebe durch Verletzung und/oder Verbrennung vorgeschädigt ist.

Typische Rahmenrezepturen für Hautbehandlungsmittel gehören zum bekannten Stand der Technik und sind beispielsweise in den Broschüren der Hersteller der jeweiligen Grund- und Wirkstoffe enthalten. Eine informative Quelle zur Art und Herstellung derartiger Formulierungen stellt beispielsweise das jährlich erscheinende Kosmetik-Jahrbuch (Herausgeber: B. Ziolkowsky, Verlag für Chemische Industrie) dar. Darüber hinaus sei auch auf NOWAK G.A., "Die kosmetischen Präparate" (beispielsweise Band 2 "Die kosmetischen Präparate - Rezeptur, Rohstoffe, wissenschaftliche Grundlagen") Verlag für Chemische Industrie, Herausgeber: Ziolkowski, verwiesen. Ebenso wird auf "The Chemistry and Manufacture of Cosmetics", Volume II - Formulating (3rd edition, Allured Publishing Corporation), Bezug genommen.

In vorteilhafter Weise können bekannte Formulierungen dermatologischer Zusammensetzungen in der Regel unverändert übernommen werden. Im Bedarfsfall können aber zur Anpassung und/oder Optimierung die gewünschten Modifizierungen durch einfache, dem Fachmann bekannte Maßnahmen vorgenommen werden.

Beispiele für erfindungsgemäße Rezepturen mit einer dermatologisch wirksamen Zusammensetzung sind nachstehend aufgeführt. Die in den nachfolgenden Tabellen aufgeführten Produkte sind erhältlich:
* ¹ = ABIL^{®} Care 85 erhältlich bei der Firma Goldschmidt GmbH
* ² = TEGINACID® C erhältlich bei der Firma Goldschmidt GmbH
* ³ = TEGIN® M erhältlich bei der Firma Goldschmidt GmbH
* ⁴ = TEGO^{®} Alkanol 1618 erhältlich bei der Firma Goldschmidt GmbH
* ⁵ = TEGO® Carbomer 134 erhältlich bei der Firma Goldschmidt GmbH
* ⁶ = TEGOSOFT® OP erhältlich bei der Firma Goldschmidt GmbH
* ⁷ = TEGO® Care PS erhältlich bei der Firma Goldschmidt GmbH
* ⁸ = TEGO® Alkanol 18 erhältlich bei der Firma Goldschmidt GmbH
* ⁹ = TEGOSOFT® liquid erhältlich bei der Firma Goldschmidt GmbH
* ¹⁰ = TEGOSOFT® CT erhältlich bei der Firma Goldschmidt GmbH
* ¹¹= TEGO® Care 450 erhältlich bei der Firma Goldschmidt GmbH
* ¹² = TEGOSOFT® OS erhältlich bei der Firma Goldschmidt GmbH
* ¹³ =TEGOSOFT® DO erhältlich bei der Firma Goldschmidt GmbH
* ¹⁴ = TEGO® Carbomer 141 erhältlich bei der Firma Goldschmidt GmbH
* ¹⁵= TEGOSOFT® PC 41 erhältlich bei der Firma Goldschmidt GmbH
* ¹⁶ = ABIL® B 8851 erhältlich bei der Firma Goldschmidt GmbH
* ¹⁷ =Euxyl K 400 erhältlich bei der Firma Schülke & Mayr
* ¹⁸ = TEGO® Carbomer 140 erhältlich bei der Firma Goldschmidt GmbH
* ¹⁹ = Macrogol 6000 erhältlich bei der Firma Sasol
* ²⁰ = HyaCare erhältlich bei der Firma Goldschmidt GmbH

### Erfindungsgemäßes Formulierungsbeispiel 1

### Oligopeptid-enthaltende pflegende Hautcreme

| | | | |
|---|---|---|---|
| A) | ABIL^{®} Care 85^{*1} | (Bis-PEG/PPG-16/16 PEG/PPG-16/16 Dimethicone; Caprylic/Capric Triglycerides) | 1,5 Gew.-% |
| | TEGINACID^{®} C^{*2} | Ceteareth-25 | 0,5 Gew.-% |
| | TEGIN^{®} M^{*3} | Glyceryl Stearate | 2,0 Gew.-% |
| | TEGO® Alkanol 1618^{*4} | Cetearyl Alcohol | 6,0 Gew.-% |
| | Cyclomethicone | | 5,0 Gew.-% |
| B) | Glycerin | | 3,0 Gew.-% |
| | Wasser | | 79,25 Gew.-% |
| | N-Octanoyl Val-Glu-Ile-Pro-Glu | | 0,005 Gew.-% |
| C) | TEGO® Carbomer 134^{*5} | Carbomer | 0,15 Gew.-% |
| | TEGOSOFT® OP^{*6} | Ethylhexyl Palmitate | 0,6 Gew.-% |
| D) | NaOH (10%) | | q. s. |
| | Parfüm | | q. s. |
| | Konservierungsmittel | | q. s. |

### Herstellung der Formulierung

Phase A) und B) werden auf 75°C erwärmt. Anschließend gibt man Phase A) unter Rühren zu B) und homogenisiert, beispielsweise mit einem Ultra Turrax. Die Emulsion wird unter leichtem Rühren bis auf 60°C abgekühlt. Bei 60°C wird Phase C) zugegeben und nochmals kurz homogenisiert. Unter Rühren wird abgekühlt und unterhalb von 40°C die Phase D) zugegeben.

### Erfindungsgemäßes Formulierungsbeispiel 2

### Oligopeptid-enthaltende O/W-Creme

| | | | |
|---|---|---|---|
| A) | TEGO® Care PS* | Methyl Glucose Sesquistearate | 3,0 Gew.-% |
| | TEGO® Alkanol 18*⁸ | Stearyl Alcohol | 1,5 Gew.-% |
| | TEGIN® M*³ | Glyceryl Stearate | 3,5.Gew.-% |
| | TEGOSOFT® liquid*⁹ | Cetearyl Ethylhexanoate | 9,2 Gew.-% |
| | TEGOSOFT® CT*¹⁰ | Caprylic/Capric Triglyceride | 10,0 Gew.-% |
| B) | Glycerin | | 3,0 Gew.-% |
| | Wasser | | 62,65 Gew.-% |
| | Oligopeptid Tyr-Pro-Ile-Pro -Phe | | 0,01 Gew.-% |
| C) | TEGO® Carbomer 134*⁵ | Carbomer | 0,2 Gew.-% |
| | TEGOSOFT® liquid*⁹ | Cetearyl-ethylhexanoate | 0,8 Gew.-% |
| | Wirkstoffkombination 1 | | 5,0 Gew.-% |
| | NaOH (10%) | | 0,65 Gew.-% |
| | Parfüm | | 0,5 Gew.-% |
| | Konservierungsmittel | | q. s. |

### Herstellung der Formulierung

Phase A) und B) werden auf 65°C erwärmt. Anschließend gibt man Phase B) zu A) und homogenisiert, beispielsweise mit einem Ultra Turrax. Die Emulsion wird unter leichtem Rühren bis auf 60°C abgekühlt. Bei 60°C wird Phase C) zugegeben und nochmals kurz homogenisiert. Die Wirkstoffkombination 1 sowie die Natronlauge und das Parfümöl werden nacheinander bei 35 bis 40°C zugegeben.

### Erfindungsgemäßes Formulierungsbeispiel 3

### O/W-Lotion mit Oligopeptid

| | | | |
|---|---|---|---|
| A) | TEGO® Care 450^{*11} | Polyglyceryl-3 Methylglucose Distearate | 2,0 Gew.-% |
| | TEGOSOFT® OS^{*12} | Ethylhexyl Stearate | 7,2 Gew.-% |
| | TEGOSOFT® DO^{*13} | Decyl Oleate | 5,0 Gew.-% |
| B) | Glycerin | | 3,0 Gew.-% |
| | Wasser | | 81,3 Gew.-% |
| | Oligopeptid Pro-Ile-Pro-Phe | | 0,1 Gew.-% |
| C) | TEGO® Carbomer 141^{*14} | Carbomer | 0,2 Gew.-% |
| | TEGOSOFT® OS^{*12} | Ethylhexyl Stearate | 0,8 Gew.-% |
| D) | NaOH (10%) | | 0,65 Gew.-% |
| E) | Parfüm, Konservierungsmittel | | q. s. |

### Herstellung der Formulierung

Phase A) und B) werden separat auf 80°C erwärmt. Anschließend gibt man Phase A) zu B) ohne Rühren und homogenisiert, beispielsweise mit einem Ultra Turrax. Die Emulsion wird unter leichtem Rühren bis auf 60°C abgekühlt. Bei 60°C wird Phase C) zugegeben und nochmals kurz homogenisiert. Die Emulsion wird unter leichtem Rühren abgekühlt bis unter 40°C. Dann wird Phase D) zugegeben.

### Erfindungsgemäßes Formulierungsbeispiel 4

### Oligopeptid-enthaltendes klares Hydrogel

| | | | |
|---|---|---|---|
| A) | TEGOSOFT® PC 41^{*15} | Polyglyceryl-4 Caprate | 1,0 Gew.-% |
| | ABIL^{®} B 8851^{*16} | PEG/PPG-14/4 Dimethicone | 7,2 Gew.-% |
| | Euxyl K 400^{*17} | Methyldibromo Glutaronitrile, Phenoxyethanol | 0,15 Gew.-% |
| | Glycerin | | 3,0 Gew.-% |
| | Parfüm | | 0,2 Gew.-% |
| B) | TEGO® Carbomer 140^{*18} | Carbomer | 0,2 Gew.-% |
| | TEGO® Carbomer 141^{*14} | Carbomer | 0,3 Gew.-% |
| | Wasser | | 49,5 Gew.-% |
| C) | Wasser | | 32,5 Gew.-% |
| | Macrogol 6000^{*19} | PEG-150^{*19} | 1,0 Gew.-% |
| | Hexanoyl-Thr-Lys-Pro-Ile-Pro-Arg | | 0,0005 Gew.-% |
| D) | NaOH (10%) | | 1,6 6 Gew.-% |
| E) | HyaCare^{*20} | Sodium Hyaluronate | 0,1 Gew.-% |
| | Wasser | | 9,9 Gew.-% |

### Herstellung der Formulierung

Die Rohstoffe der Phase A) werden in der angegebenen Reihenfolge gemischt. Anschließend gibt man Phase B) unter Rühren hinzu. Danach wird Phase C) unter Rühren zugegeben. Danach wird Phase D) unter Rühren zugegeben. Abschließend wird Phase E) zugegeben und so lange gerührt, bis die Formulierung klar ist.

Die hautpharmakologische Wirkung der erfindungsgemäßen Peptide wird in einer *"in vivo* Testreihe" demonstriert:
Für die nachfolgend beschriebenen Wirksamkeitsstudien wird eine einfach aufgebaute O/W-Creme eingesetzt, die folgende Zusammensetzung aufweist:

| Oligo-Peptid enthaltende dermatologische Zusammensetzung | |
|---|---|
| Ceteareth-25 | 2,0 Gew.-% |
| Glyceryl Stearate | 4,0 Gew.-% |
| Stearyl Alcohol | 2,0 Gew.-% |
| Ethylhexyl Stearate | 8,5 Gew.-% |
| Caprylic/Capric Triglyceride | 8,5 Gew.-% |
| Konservierungsmittel | 0,1 Gew.-% |
| Wasser | 74,8 Gew.-% |
| Oligopeptid Val-Glu-Ile-Pro-Glu | 0,1 Gew.-% |

Für den Test werden zehn freiwillige Personen ausgewählt. Jede Person appliziert über einen Zeitraum von 10 Tagen zweimal täglich eine definierte Menge der Oligo-Peptid enthaltenden O/W-Creme auf dem linken volaren Unterarm auf. Als Kontrolle wird eine äquivalente Menge Basis-Creme ohne Oligopeptid-Zusatz auf dem rechten Unterarm aufgetragen. An jede dieser Personen wird eine Wärmesonde mit einem eingebauten Thermometer an jedem Oberarm angelegt, mit der sich ein abgegrenzter Bereich der Haut erwärmen und anschließend die Temperatur messen lässt. Die Personen geben an, wann die vorab definierte Schwelle für "lauwarm", "warm", "sehr heiß" und "schmerzhaft" überschritten wird. Nach Feststellung dieser Empfindungen und ihrer jeweiligen Empfindungsschwellen tragen die Personen die dermatologische Zusammensetzung, die das zu testende erfindungsgemäße Oligopeptid enthält, im Messbereich auf die Haut auf. Nach unterschiedlichen Wartezeiten werden die Schwellenwerte für das Wärmeempfinden erneut auf die gleiche Weise bestimmt. Auf diese Weise lässt sich eine Zunahme der Hautempfindlichkeit bzw. Steigerung der neuronalen Wahrnehmung der Haut im Vergleich mit der gleichen dermatologischen Zusammensetzung testen, die das Oligopeptid nicht enthält.

Die Wirksamkeit der erfindungsgemäßen Oligopeptide werden in diesem Test nachgewiesen: eine Zunahme der Wärmeempfindlichkeit an dem mit der der Oligo-Peptid enthaltenden O/W-Creme behandelten linken Unterarm wird nach 30 Minuten bemerkbar und wird nach 2 Stunden deutlich.

Es kann mit diesem Test nachgewiesen werden, dass die Hautempfindlichkeit und Reizwahrnehmung durch die erfindungsgemäße dermatologische Zusammensetzung gesteigert werden kann und diese geeignet ist, die Hautsensorik- und -wahrnehmung zu stimulieren.

Die dermatologischen Zusammensetzungen können beispielsweise in der medizinischen Behandlung von Narbengewebe oder begleitend in der Therapie von Patienten eingesetzt werden, die beispielsweise durch einen Schlaganfall das Wahrnehmungsvermögen für Reize über die Haut verloren haben oder deren Hautempfindlichkeit durch andere Schädigungen oder Vorerkrankungen reduziert oder gestört ist.

Die dermatologischen Zusammensetzungen können beispielsweise auch zur kosmetischen Behandlung von Alterungsphänomenen der Haut verwendet werden und einer Verminderung ihrer funktionstragenden Komponente, wie der reduzierten neuronalen Wahrnehmung der Haut, entgegenwirken. Durch die Applikation erfindungsgemäßer dermatologischer Zusammensetzungen kann das Hautgefühl und damit auch das Allgemeinbefinden deutlich verbessert und positiv beeinflusst werden.

## Patentansprüche

1. Dermatologische Zusammensetzungen enthaltend mindestens ein die Sequenz Ala-IIe-Pro-Gln, Arg-Lys-Pro-Ile-Pro, Palmitoyl-Gly-His-Pro-Ile-Ser, Gly-Pro-Ile-Pro, Gly-Val-Glu-Ile-Pro-Ala, Pro-Ile-Pro-Phe, Octanoyl-Ser-His-Pro-Ile, Hexanoyl-Thr-Lys-Pro-Ile-Pro-Arg, Tyr-Pro-Ile-Ser-Leu, Val-Glu-Ile-Pro-Glu oder Tyr-Pro-Ile-Pro-Phe aufweisendes Oligopeptid und/oder ein Acyl-Derivat hiervon zur Verwendung als Mittel zur Steigerung der Hautempfindlichkeit bei reduzierter oder verlorener Wahrnehmungskraft über das Sinnesorgan Haut.

2. Dermatologische Zusammensetzungen zur Verwendung nach Anspruch 1, **dadurch gekennzeichnet, dass** das Oligopeptid mit substituierten oder unsubstituierten Acetyl-, Hexanoyl-, Myristyl-, Palmitoyl- und/oder Biotinyl-Gruppen derivatisiert ist.

3. Dermatologische Zusammensetzungen zur Verwendung nach Anspruch 1 oder 2, **dadurch gekennzeichnet, dass** das Oligopeptid N-Palmitoyl-Val-Glu-Ile-Pro-Glu und/oder N-Palmitoyl-Tyr-Pro-Ile-Pro-Phe ist.

4. Dermatologische Zusammensetzungen zur Verwendung nach einem der Ansprüche 1 bis 3, **dadurch gekennzeichnet, dass** das Oligopeptid in einer Konzentration enthalten ist, die zwischen 0,0005 Ges.-% und 2 Gew.-%, bevorzugt zwischen 0,01 Gew.-% und 1 Gew.-%, und weiter bevorzugt zwischen 0,05 Gew.-% und 0,5 Ges.-%, bezogen auf das Gewicht der Gesamtzusammensetzung, liegt.

5. Dermatologische Zusammensetzungen zur Verwendung nach einem der Ansprüche 1 bis 4, **dadurch gekennzeichnet, dass** ergänzend ein oder mehrere dermatologisch verträgliche Zusatz- und/oder Hilfsstoffe enthalten sind, wobei vorzugsweise mindestens einer davon eine Trägersubstanz ist.

6. Dermatologische Zusammensetzungen zur Verwendung nach einem der Ansprüche 1 bis 5, **dadurch gekennzeichnet, dass** das Oligopeptid zusammen mit einem oder mehreren weiteren pharmazeutisch und/oder kosmetisch wirksamen Substanzen enthalten ist.

7. Dermatologische Zusammensetzungen zur Verwendung nach einem der Ansprüche 1 bis 6, **dadurch gekennzeichnet, dass** es sich um ein Arzneimittel und/oder eine kosmetische Zusammensetzung handelt.

8. Verwendung mindestens eines die Sequenz Ala-IIe-Pro-Gln, Arg-Lys-Pro-Ile-Pro, Palmitoyl-Gly-His-Pro-Ile-Ser, Gly-Pro-Ile-Pro, Gly-Val-Glu-Ile-Pro-Ala, Pro-Ile-Pro-Phe, Octanoyl-Ser-His-Pro-Ile, Hexanoyl-Thr-Lys-Pro-Ile-Pro-Arg, Tyr-Pro-Ile-Ser-Leu, Val-Glu-Ile-Pro-Glu oder Tyr-Pro-Ile-Pro-Phe aufweisenden Oligopeptids und/oder eines Acyl-Derivats hiervon zur Herstellung einer dermatologischen Zusammensetzung nach einem der Ansprüche 1 bis 7 zur Steigerung der Hautempfindlichkeit bei reduzierter oder verlorener Wahrnehmungskraft über das Sinnesorgan Haut.

## Claims

1. Dermatological compositions comprising at least one oligopeptide having the sequence Ala-IIe-Pro-GIn, Arg-Lys-Pro-Ile-Pro, palmitoyl-Gly-His-Pro-Ile-Ser, Gly-Pro-Ile-Pro, Gly-Val-Glu-Ile-Pro-Ala, Pro-Ile-Pro-Phe, octanoyl-Ser-His-Pro-Ile, hexanoyl-Thr-Lys-Pro-Ile-Pro-Arg, Tyr-Pro-Ile-Ser-Leu, Val-Glu-Ile-Pro-Glu or Tyr-Pro-Ile-Pro-Phe and/or an acyl derivative thereof for use as a means for increasing skin sensitivity in cases of reduced or lost power of perception via the sensory organ skin.

2. Dermatological compositions for use according to Claim 1, **characterized in that** the oligopeptide is derivatized with substituted or unsubstituted acetyl, hexanoyl, myristyl, palmitoyl and/or biotinyl groups.

3. Dermatological compositions for the use according to Claim 1 or 2, **characterized in that** the oligopeptide is N-palmitoyl-Val-Glu-Ile-Pro-Glu and/or N-palmitoyl-Tyr-Pro-Ile-Pro-Phe.

4. Dermatological compositions for the use according to one of Claims 1 to 3, **characterized in that** the oligopeptide is present in a concentration between 0.0005% by weight and 2% by weight, preferably between 0.01% by weight and 1% by weight, and further preferably between 0.05% by weight and 0.5% by weight, based on the weight of the total composition.

5. Dermatological compositions for the use according to one of Claims 1 to 4, **characterized in that** additionally one or more dermatologically compatible additives and/or auxiliaries are present, where preferably at least one of them is a carrier substance.

6. Dermatological compositions for the use according to one of Claims 1 to 5, **characterized in that** the oligopeptide is present together with one or more further pharmaceutically and/or cosmetically effective substances.

7. Dermatological compositions for the use according to one of Claims 1 to 6, **characterized in that** it is a medicament and/or a cosmetic composition.

8. Use at least of one oligopeptide having the sequence Ala-IIe-Pro-Gln, Arg-Lys-Pro-Ile-Pro, palmitoyl-Gly-His-Pro-Ile-Ser, Gly-Pro-Ile-Pro, Gly-Val-Glu-Ile-Pro-Ala, Pro-Ile-Pro-Phe, octanoyl-Ser-His-Pro-Ile, hexanoyl-Thr-Lys-Pro-Ile-Pro-Arg, Tyr-Pro-Ile-Ser-Leu, Val-Glu-Ile-Pro-Glu or Tyr-Pro-Ile-Pro-Phe and/or of an acyl derivative thereof for the preparation of a dermatological composition according to one of Claims 1 to 7 for increasing skin sensitivity in cases of reduced or lost power of perception via the sensory organ skin.

## Revendications

1. Compositions dermatologiques, contenant au moins un oligopeptide présentant la séquence Ala-IIe-Pro-Gln, Arg-Lys-Pro-Ile-Pro, palmitoyl-Gly-His-Pro-Ile-Ser, Gly-Pro-Ile-Pro, Gly-Val-Glu-Ile-Pro-Ala, Pro-Ile-Pro-Phe, octanoyl-Ser-His-Pro-Ile, hexanoyl-Thr-Lys-Pro-Ile-Pro-Arg, Tyr-Pro-Ile-Ser-Leu, Val-Glu-Ile-Pro-Glu ou Tyr-Pro-Ile-Pro-Phe et/ou un dérivé d'acyle de celui-ci destinées à une utilisation comme agent pour augmenter la sensibilité de la peau dans le cas d'une force de perception réduite ou perdue via l'organe de sens peau.

2. Compositions dermatologiques destinées à une utilisation selon la revendication 1, **caractérisées en ce que** l'oligopeptide est dérivé avec des groupes acétyle, hexanoyle, myristyle, palmitoyle et/ou biotinyle substitués ou non substitués.

3. Compositions dermatologiques destinées à une utilisation selon la revendication 1 ou 2, **caractérisées en ce que** l'oligopeptide est le N-palmitoyl-Val-Glu-Ile-Pro-Glu et/ou le N-palmitoyl-Tyr-Pro-Ile-Pro-Phe.

4. Compositions dermatologiques destinées à une utilisation selon l'une quelconque des revendications 1 à 3, **caractérisées en ce que** l'oligopeptide est contenu en une concentration qui est située entre 0,0005% en poids et 2% en poids, de préférence entre 0,01% en poids et 1% en poids, et plus préférablement entre 0,05% en poids et 0,5% en poids, par rapport au poids de la composition totale.

5. Compositions dermatologiques destinées à une utilisation selon l'une quelconque des revendications 1 à 4, **caractérisées en ce qu'**elles contiennent à titre complémentaire un ou plusieurs additifs et/ou adjuvants dermatologiquement compatibles, où de préférence au moins l'un est une substance support.

6. Compositions dermatologiques destinées à une utilisation selon l'une quelconque des revendications 1 à 5, **caractérisées en ce que** l'oligopeptide est contenu ensemble avec une ou plusieurs autres substances pharmaceutiquement et/ou cosmétiquement actives.

7. Compositions dermatologiques destinées à une utilisation selon l'une quelconque des revendications 1 à 6, **caractérisées en ce qu'**il s'agit d'un médicament et/ou d'une composition cosmétique.

8. Utilisation d'au moins un oligopeptide présentant la séquence Ala-IIe-Pro-Gln, Arg-Lys-Pro-Ile-Pro, palmitoyl-Gly-His-Pro-Ile-Ser, Gly-Pro-Ile-Pro, Gly-Val-Glu-Ile-Pro-Ala, Pro-Ile-Pro-Phe, octanoyl-Ser-His-Pro-Ile, hexanoyl-Thr-Lys-Pro-Ile-Pro-Arg, Tyr-Pro-Ile-Ser-Leu, Val-Glu-Ile-Pro-Glu ou Tyr-Pro-Ile-Pro-Phe et/ou d'un dérivé d'acyle de celui-ci pour la préparation d'une composition dermatologique selon l'une quelconque des revendications 1 à 7 destinée à augmenter la sensibilité de la peau dans le cas d'une force de perception réduite ou perdue via l'organe de sens peau.
